# FASCICULE DE BREVET EUROPEEN

(11) **EP 4 070 848 B1**
(45) Date de publication et mention de la délivrance du brevet: **12.02.2025**
(21) Numéro de dépôt: 22160696.5
(22) Date de dépôt: 08.03.2022
(51) Int. Cl.: A61M 60/196, A61M 39/10, A61M 60/268, A61M 60/835, A61M 60/859

(54) **DISPOSITIF DE RACCORDEMENT D'UNE PROTHÈSE CARDIAQUE IMPLANTABLE AU RÉSEAU VASCULAIRE D'UN PATIENT ET PROTHÈSE CARDIAQUE POURVUE D'UN TEL DISPOSITIF DE RACCORDEMENT**
VORRICHTUNG ZUM VERBINDEN EINER IMPLANTIERBAREN HERZPROTHESE MIT DEM GEFÄSSNETZ EINES PATIENTEN UND HERZPROTHESE, DIE EINE SOLCHE VERBINDUNGSVORRICHTUNG UMFASST
DEVICE FOR CONNECTING AN IMPLANTABLE CARDIAC PROSTHESIS IN THE VASCULAR SYSTEM OF A PATIENT AND CARDIAC PROSTHESIS EQUIPPED WITH SUCH A CONNECTION DEVICE

(30) Priorité: 06.04.2021 FR 2103482
(43) Date de publication de la demande: 12.10.2022
(73) Titulaire: CARMAT, 78140 Vélizy Villacoublay (FR)
(72) Inventeur: Parra d'Andert, Foulques, 91190 Gif-sur-Yvette (FR); Grimmé, Marc, 91120 Palaiseau (FR); Gourmault, Pascal, 63200 Riom (FR); Dubuis, Pascal, 63720 Clerlande (FR)
(74) Mandataire: Gevers & Orès

(56) Documents cités:
- FR-B1- 2 784 585
- US-A1- 2009 192 607
- US-A1- 2009 276 041

## Description

### DOMAINE TECHNIQUE

La présente invention concerne un dispositif de raccordement destiné au raccordement d'une prothèse cardiaque au réseau vasculaire d'un patient, ladite prothèse cardiaque étant implantable dans la cavité péricardique du patient et étant apte à remplacer les ventricules gauche et droit naturels dudit patient après leur ablation. La présente invention concerne également une prothèse cardiaque pourvue d'un tel dispositif de raccordement.

### ÉTAT DE LA TECHNIQUE

Une prothèse cardiaque totalement implantable, telle que décrite par exemple dans les brevets FR-2 784 585 et FR-2 902 345, a pour fonction de remplacer les ventricules du patient en conservant les oreillettes. Pour ce faire, elle comporte un corps de prothèse rigide dans lequel sont notamment agencés des ventricules artificiels.

Pour l'implantation de cette prothèse cardiaque dans la cavité péricardique du patient, il est donc nécessaire de créer des interfaces entre le réseau vasculaire (oreillettes et artères) du patient et la prothèse cardiaque en respectant l'anatomie du patient. Pour cela, il faut notamment prendre en compte :
- une orientation définie des oreillettes (gauche et droite) avec un écartement plus ou moins constant ; et
- une orientation et une disposition pouvant être très variables des artères (aorte et artère pulmonaire).

Une solution utilisée actuellement comprend, d'une part, une pièce d'interface (correspondant à un système de lunettes) pour les admissions de la prothèse cardiaque, et d'autre part, deux conduits souples (ou conduits vasculaires) pour les éjections de la prothèse cardiaque.

Les brevets FR-2 902 343 et FR-2 902 344 décrivent un système de lunettes coopérantes, comme interface d'admission, pour les admissions d'une prothèse cardiaque. Ce système de lunettes comprend une première lunette faisant partie intégrante du corps de prothèse rigide de la prothèse cardiaque et comportant des premier et deuxième orifices, respectivement en communication avec le ventricule artificiel gauche et avec le ventricule artificiel droit de la prothèse cardiaque, et une seconde lunette comportant des troisième et quatrième orifices aptes à être respectivement reliés à l'oreillette naturelle gauche et à l'oreillette naturelle droite du patient via des collerettes qui sont suturées sur les oreillettes. Ces première et seconde lunettes sont configurées pour pouvoir être raccordées l'une à l'autre de façon amovible, avec les premier et troisième orifices en regard et les deuxième et quatrième orifices en regard. Ce système de lunettes facilite le raccordement de la prothèse cardiaque (pourvue de la première lunette) à la seconde lunette (préalablement raccordée aux oreillettes naturelles).

La mise en place de chacune des lunettes de cette interface d'admission est réalisée avant la mise en place de la prothèse cardiaque. En revanche, les conduits vasculaires sont d'abord montés sur la prothèse cardiaque, via des agrafes, avant d'être ensuite suturés sur les artères du patient. La présence de la prothèse cardiaque offre un espace réduit d'intervention pour le chirurgien et donc un accès difficile pour réaliser les sutures. La mise en place finale de la prothèse cardiaque est réalisée par clipsage de la prothèse cardiaque sur l'interface d'admission.

La présence de la prothèse cardiaque lors des sutures des conduits vasculaires sur les artères pose un problème d'ergonomie. En effet, la prothèse cardiaque doit être maintenue par une personne autre que le chirurgien réalisant les sutures et l'accès aux artères est limité. Ainsi, deux personnes sont nécessaires pour la mise en place.

Par ailleurs, le système de fixation des conduits vasculaires nécessite l'utilisation de plusieurs ancillaires d'implantation et le montage est difficile (montage serré) en raison des exigences d'étanchéité.

### EXPOSE DE L'INVENTION

La présente invention, dont l'étendue est limitée par le jeu de revendications, a pour objet de trouver une solution permettant de simplifier et d'améliorer l'opération d'implantation de la prothèse cardiaque en permettant au chirurgien de travailler dans un environnement moins contraint pour la mise en place et les sutures. Pour ce faire, elle concerne un dispositif de raccordement destiné au raccordement d'une prothèse cardiaque implantable dans la cavité péricardique d'un patient au réseau vasculaire du patient, ladite prothèse cardiaque étant apte à remplacer les ventricules gauche et droit naturels du patient après ablation de ces derniers.

Selon l'invention, ledit dispositif de raccordement comporte une unique pièce d'interface configurée pour pouvoir être fixée à la prothèse cardiaque, la pièce d'interface étant munie d'un élément d'interface dit mitral, d'un élément d'interface dit tricuspide, d'un élément d'interface dit aortique et d'un élément d'interface dit pulmonaire destinés, respectivement, au raccordement à l'oreillette gauche, à l'oreillette droite, à l'aorte et à l'artère pulmonaire du patient, chacun desdits éléments d'interface étant pourvu d'un orifice, et lesdits éléments d'interface présentant des orientations prédéterminées (appropriées).

Ainsi, grâce à l'invention, le dispositif de raccordement pourvu d'une pièce d'interface unique comprenant l'ensemble des éléments d'interface avec, de plus, des orientations appropriées de ces éléments d'interface comme précisé ci-dessous, permet de simplifier et de faciliter l'opération de mise en place de la prothèse cardiaque en permettant au chirurgien de travailler dans un environnement moins contraint pour la mise en place et les opérations de suture.

Comme également précisé ci-dessous, ledit dispositif de raccordement apporte beaucoup d'autres avantages, et notamment les avantages suivants :
- une amélioration de l'ergonomie du chirurgien ;
- une réduction du nombre de pièces implantées et du nombre de pièces nécessaires à l'implantation ; et
- une réduction de la durée d'implantation.

Dans le cadre de la présente invention, les orientations relatives des éléments d'interface de la pièce d'interface sont adaptées à la fois aux caractéristiques de la prothèse cardiaque et à une anatomie donnée, notamment une anatomie moyenne, de patients aptes à recevoir la prothèse cardiaque, la prothèse cardiaque étant conçue pour respecter cette anatomie.

De plus, les positions (et distances) relatives des éléments d'interface de la pièce d'interface sont également adaptées à la fois aux caractéristiques de la prothèse cardiaque et à une anatomie donnée, notamment une anatomie moyenne, de patients aptes à recevoir la prothèse cardiaque.

Ainsi, avantageusement, l'orientation de l'élément d'interface mitral et l'orientation de l'élément d'interface tricuspide présentent entre elles un angle compris entre 0 °et 32°, et de préférence entre 0° et 20°. De plus, dans un mode de réalisation particulier, l'élément d'interface mitral et l'élément d'interface tricuspide sont agencés sur une plaque plane de la pièce d'interface, ce qui permet notamment de faciliter la réalisation.

En outre, avantageusement, la pièce d'interface présente au moins certaines des orientations suivantes :
- l'orientation de l'élément d'interface aortique et l'orientation de l'élément d'interface mitral présentent, entre elles, un angle compris entre 0 °et 90°, et de préférence entre 20° et 45° ;
- l'orientation de l'élément d'interface aortique et l'orientation de l'élément d'interface tricuspide présentent, entre elles, un angle compris entre 0° et 90°, et de préférence entre 20° et 45° ;
- l'orientation de l'élément d'interface pulmonaire et l'orientation de l'élément d'interface mitral présentent, entre elles, un angle compris entre 26° et 64°, et de préférence entre 40° et 60° ;
- l'orientation de l'élément d'interface pulmonaire et l'orientation de l'élément d'interface tricuspide présentent, entre elles, un angle compris entre 31° et 73°, et de préférence entre 40° et 60° ;
- l'orientation de l'élément d'interface aortique et l'orientation de l'élément d'interface pulmonaire présentent, entre elles, un angle compris entre 39° et 79°, et de préférence entre 55° et 70°.

De plus, avantageusement, la pièce d'interface présente au moins certaines des distances suivantes :
- la distance entre le centre de l'orifice de l'élément d'interface mitral et le centre de l'orifice de l'élément d'interface tricuspide est comprise entre 42 mm et 76 mm, et de préférence entre 45 mm et 60 mm ;
- la distance entre le centre de l'orifice de l'élément d'interface mitral et le centre de l'orifice de l'élément d'interface aortique est comprise entre 37 mm et 65 mm, et de préférence entre 40 mm et 55 mm ;
- la distance entre le centre de l'orifice de l'élément d'interface mitral et le centre de l'orifice de l'élément d'interface pulmonaire est comprise entre 50 mm et 80 mm, et de préférence entre 60 mm et 70 mm ;
- la distance entre le centre de l'orifice de l'élément d'interface tricuspide et le centre de l'orifice de l'élément d'interface aortique est comprise entre 34 mm et 64 mm, et de préférence entre 35 mm et 50 mm ;
- la distance entre le centre de l'orifice de l'élément d'interface tricuspide et le centre de l'orifice de l'élément d'interface pulmonaire est comprise entre 59 mm et 91 mm, et de préférence entre 70 mm et 80 mm ;
- la distance entre le centre de l'orifice de l'élément d'interface aortique et le centre de l'orifice de l'élément d'interface pulmonaire est comprise entre 22 mm et 46 mm, et de préférence entre 30 mm et 40 mm.

Dans un mode de réalisation préféré, la pièce d'interface est réalisée en l'un des matériaux suivants : du titane, de l'inox.

En outre, de façon avantageuse, l'élément d'interface aortique et l'élément d'interface pulmonaire comportent, chacun, un filetage, pour permettre un vissage, comme précisé ci-dessous.

Dans un mode de réalisation préféré, le dispositif de raccordement comporte également :
- un premier conduit vasculaire dont une première extrémité est destinée à être suturée sur l'aorte et dont la seconde extrémité est munie d'une bague montée de façon libre en rotation et apte à être vissée sur l'élément d'interface aortique ; et/ou
- un second conduit vasculaire dont une première extrémité est destinée à être suturée sur l'artère pulmonaire et dont la seconde extrémité est munie d'une bague montée de façon libre en rotation et apte à être vissée sur l'élément d'interface pulmonaire ; et/ou
- une première collerette de suture destinée à être suturée sur l'oreillette gauche et comportant une âme configurée pour pouvoir être montée sur l'élément d'interface mitral ; et/ou
- une seconde collerette de suture destinée à être suturée sur l'oreillette droite du patient et comportant une âme configurée pour pouvoir être montée sur l'élément d'interface tricuspide.

La présente invention concerne également une prothèse cardiaque implantable dans la cavité péricardique d'un patient, ladite prothèse cardiaque étant apte à remplacer les ventricules gauche et droit naturels du patient après ablation de ces derniers.

Selon l'invention, la prothèse cardiaque comporte au moins un corps de prothèse, dans lequel sont agencés au moins des ventricules artificiels gauche et droit, ainsi qu'un dispositif de raccordement tel que celui décrit ci-dessus, qui est configuré pour pouvoir être fixé dans un logement prévu dans le corps de prothèse.

Avantageusement, la pièce d'interface du dispositif de raccordement comporte une languette de centrage qui coopère avec une gorge pratiquée dans la paroi externe du corps de prothèse, pour faciliter la mise en place de la prothèse sur le dispositif de raccordement.

Après sa mise en place dans le logement, le dispositif de raccordement est fixé sur le corps de prothèse. Dans le cadre de la présente invention, différents moyens de fixation sont envisageables pour une fixation amovible de la pièce d'interface sur le corps de prothèse.

Dans un premier mode de réalisation, la prothèse cardiaque comporte des moyens de fixation par vissage, tandis que, dans un second mode de réalisation, la prothèse cardiaque comporte des moyens de fixation par clipsage.

### BRÈVE DESCRIPTION DES FIGURES

Les figures annexées feront bien comprendre comment l'invention peut être réalisée. Sur ces figures, des références identiques désignent des éléments semblables.
La figure 1 est une vue en perspective d'une face externe d'une pièce d'interface d'un dispositif de raccordement.
La figure 2 est une vue schématique, en perspective, d'une partie d'une prothèse cardiaque pourvue d'un dispositif de raccordement.
La figure 3 est une figure similaire à celle de la figure 1 présentant différentes caractéristiques d'orientation et de distance d'éléments d'interface de la pièce d'interface.
La figure 4 est une vue en perspective d'une face interne d'une pièce d'interface d'un dispositif de raccordement, pourvue de conduits vasculaires représentés partiellement.
La figure 5 est une vue en perspective de la face interne de la pièce d'interface de la figure 1, montrant différents plans d'appui.
La figure 6 est une vue schématique, en perspective, d'une partie d'un corps de prothèse d'une prothèse cardiaque, pourvu d'un logement destiné à recevoir un dispositif de raccordement.
La figure 7 est une vue en perspective d'un conduit vasculaire d'un dispositif de raccordement.
La figure 8 est une vue en perspective d'une collerette de suture d'un dispositif de raccordement.
La figure 9 est une vue schématique, en perspective, de moyens de fixation par vissage.
La figure 10 est une vue schématique latérale de moyens de fixation par clipsage.

### DESCRIPTION DÉTAILLÉE

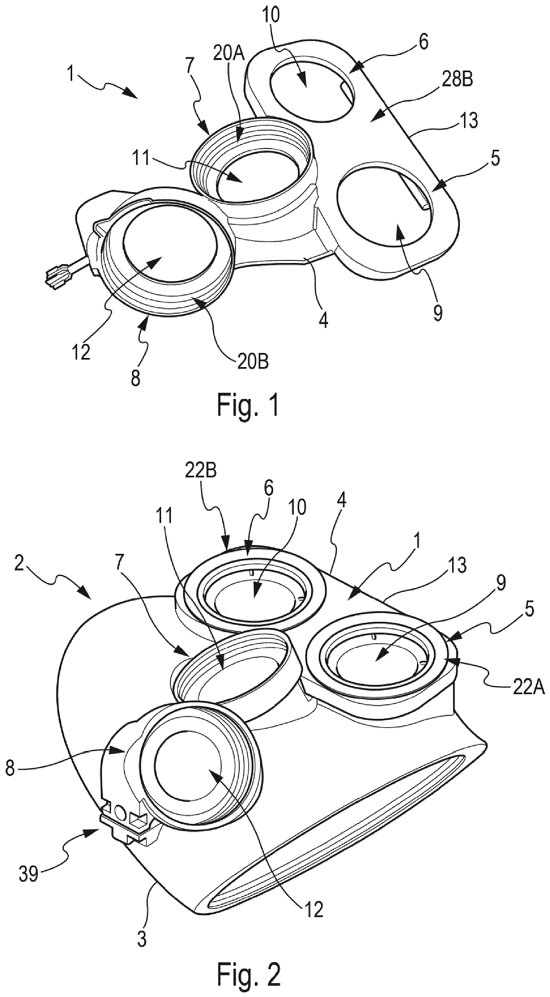

Le dispositif de raccordement 1 illustrant l'invention et représenté schématiquement dans un mode de réalisation particulier sur la figure 1 est destiné au raccordement d'une prothèse cardiaque 2 au réseau vasculaire (non représenté) d'un patient.

Cette prothèse cardiaque 2 représentée partiellement sur la figure 2 est implantable dans la cavité thoracique et péricardique du patient et est apte à remplacer ses ventricules gauche et droit naturels, après leur ablation.

Pour ce faire, la prothèse cardiaque 2 comporte un corps de prothèse 3 rigide, dans lequel sont notamment agencés des ventricules gauche et droit artificiels (non représentés) destinés à remplacer les ventricules gauche et droit naturels du patient.

La prothèse cardiaque 2 comporte tous les moyens nécessaires à son fonctionnement. En particulier, elle peut comporter au moins certaines des caractéristiques présentées dans les brevets FR-2 784 585 et FR-2 902 345 précités. La prothèse cardiaque 2, et notamment les éléments agencés dans le corps de prothèse 3, ne sont pas décrits davantage dans la description suivante.

Le dispositif de raccordement 1 est destiné au raccordement de la prothèse cardiaque 2 à l'oreillette gauche (naturelle), à l'oreillette droite (naturelle), à l'aorte (naturelle) et à l'artère pulmonaire (naturelle) du patient.

Pour ce faire, ledit dispositif de raccordement 1 comporte, selon l'invention, comme représenté sur la figure 1, une (unique) pièce d'interface 4 configurée pour pouvoir être fixée au corps de prothèse 3 de la prothèse cardiaque 2.

Cette pièce d'interface 4 est une pièce rigide et échancrée. Elle reprend les orientations de la prothèse cardiaque 2 et respecte l'anatomie du patient, comme précisé ci-dessous.

Pour ce faire, la pièce d'interface 4 est pourvue de quatre éléments d'interface, à savoir comme représenté sur la figure 1 (montrant une face externe 28B de la pièce d'interface 4) :
- d'un élément d'interface dit mitral 5, destiné au raccordement de la prothèse cardiaque à l'oreillette gauche (naturelle) du patient ;
- d'un élément d'interface dit tricuspide 6, destiné au raccordement de la prothèse cardiaque à l'oreillette droite (naturelle) du patient ;
- d'un élément d'interface dit aortique 7, destiné au raccordement de la prothèse cardiaque à l'aorte (naturelle) du patient ; et
- d'un élément d'interface dit pulmonaire 8, destiné au raccordement de la prothèse cardiaque à l'artère pulmonaire (naturelle) du patient.

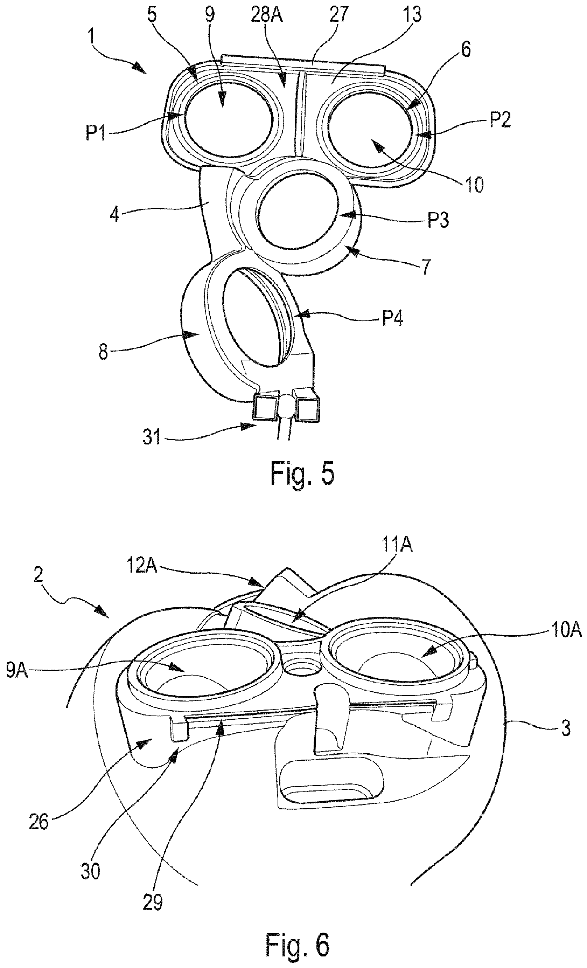

Par conséquent, la pièce d'interface 4 unique permet de réaliser la liaison avec la prothèse cardiaque 2 pour l'ensemble de ses quatre accès (deux en admission et deux en éjection) illustrés par des ouvertures 9A à 12A sur la figure 6, pour accéder aux ventricules gauche et droit artificiels de la prothèse cardiaque 2.

De plus, selon l'invention, chacun desdits éléments d'interface 5 à 8 est pourvu d'un orifice 9 à 12, et au moins certains desdits éléments d'interface 5 à 8 présentent des orientations différentes, comme précisé ci-dessous.

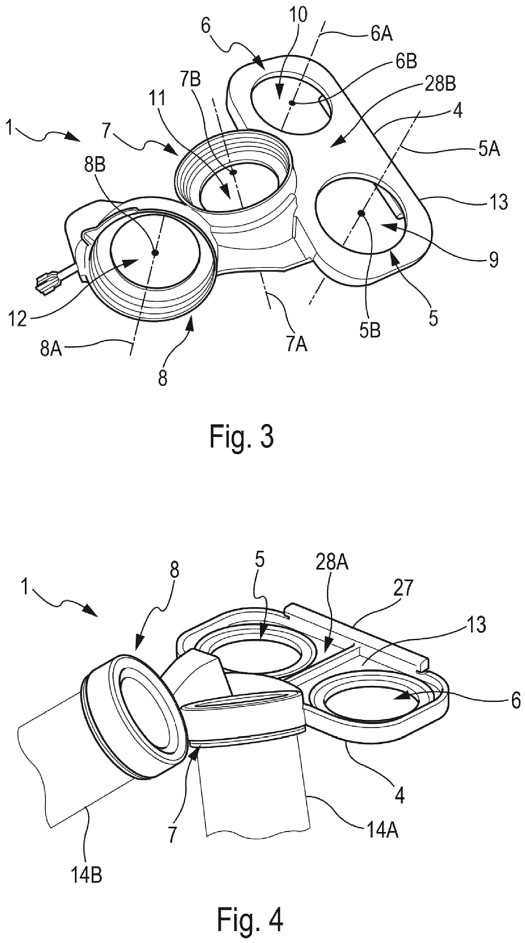

On entend par « orientation » la direction de l'axe de l'orifice de l'élément d'interface, comme représenté sur la figure 3 par des axes 5A, 6A, 7A et 8A respectivement pour les orifices 9, 10, 11 et 12 des éléments d'interface 5, 6, 7 et 8.

Les orientations peuvent également être définies par des plans d'interface ou plans d'appui P1, P2, P3 et P4 précisés ci-dessous, qui sont orthogonaux auxdits axes 5A, 6A, 7A et 8A.

Les orientations des éléments d'interface 5 à 8 sont adaptées à la fois aux caractéristiques techniques de la prothèse cardiaque 2 et à une anatomie moyenne des patients potentiels, notamment pour simplifier la mise en place de la prothèse cardiaque 2.

Dans le mode de réalisation particulier représenté, notamment sur la figure 3, l'élément d'interface mitral 5 et l'élément d'interface tricuspide 6 présentent la même orientation. Les axes 5A et 6A sont parallèles. De plus, l'élément d'interface mitral 5 et l'élément d'interface tricuspide 6 sont formés dans une seule et même plaque plane 13 de la pièce d'interface 4, qui va être positionnée dans le plan auriculaire du patient.

Toutefois, dans le cadre de la présente invention, l'orientation 5A de l'élément d'interface mitral 5 et l'orientation 6A de l'élément d'interface tricuspide 6 peuvent présenter, entre elles, un angle non nul. Plus généralement, elles peuvent présenter, entre elles, un angle compris entre 0 °et 32°, et de préférence entre 0° et 20°.

En outre, l'orientation 7A de l'élément d'interface aortique 7 et l'orientation 5A de l'élément d'interface mitral 5 présentent, entre elles, un angle compris entre 0° et 90°, et de préférence entre 20° et 45°.

De même, l'orientation 7A de l'élément d'interface aortique 7 et l'orientation 6A de l'élément d'interface tricuspide 6 présentent, entre elles, un angle compris entre 0°et 90°, et de préférence entre 20° et 45°.

En outre, l'orientation 8A de l'élément d'interface pulmonaire 8 et l'orientation 5A de l'élément d'interface mitral 5 présentent, entre elles, un angle compris entre 26° et 64°, et de préférence entre 40° et 60°.

De même, l'orientation 8A de l'élément d'interface pulmonaire 8 et l'orientation 6A de l'élément d'interface tricuspide 6 présentent, entre elles, un angle compris entre 31° et 73°, et de préférence entre 40° et 60°.

Par ailleurs, l'orientation 7A de l'élément d'interface aortique 7 et l'orientation 8A de l'élément d'interface pulmonaire 8 présentent, entre elles, un angle compris entre 39° et 79°, et de préférence entre 55° et 70°.

Les orientations des orifices (ou des plans d'interface) sont définies par la conception de la prothèse cardiaque 2 et sont issues de données physiologiques de patients.

Par ailleurs, à titre d'illustration, dans l'exemple particulier de la figure 3, on peut prévoir les distances suivantes entre les centres 5B à 8B des différents orifices 9 à 12 de la pièce d'interface 4 :
- entre 42 mm et 76 mm et de préférence entre 45 mm et 60 mm, pour la distance entre le centre 5B de l'orifice 9 de l'élément d'interface mitral 5 et le centre 6B de l'orifice 10 de l'élément d'interface tricuspide 6 ;
- entre 37 mm et 65 mm et de préférence entre 40 mm et 55 mm, pour la distance entre le centre 5B de l'orifice 9 de l'élément d'interface mitral 5 et le centre 7B de l'orifice 11 de l'élément d'interface aortique 7 ;
- entre 50 mm et 80 mm et de préférence entre 60 mm et 70 mm, pour la distance entre le centre 5B de l'orifice 9 de l'élément d'interface mitral 5 et le centre 8B de l'orifice 12 de l'élément d'interface pulmonaire 8 ;
- entre 34 mm et 64 mm et de préférence entre 35 mm et 50 mm, pour la distance entre le centre 6B de l'orifice 10 de l'élément d'interface tricuspide 6 et le centre 7B de l'orifice 11 de l'élément d'interface aortique 7 ;
- entre 59 mm et 91 mm et de préférence entre 70 mm et 80 mm, pour la distance entre le centre 6B de l'orifice 10 de l'élément d'interface tricuspide 6 et le centre 8B de l'orifice 12 de l'élément d'interface pulmonaire 8 ; et
- entre 22 mm et 46 mm et de préférence entre 30 mm et 40 mm, pour la distance entre le centre 7B de l'orifice de l'élément d'interface aortique 7 et le centre 8B de l'orifice 12 de l'élément d'interface pulmonaire 8.

Dans un mode de mise en œuvre préféré, les distances et les angles entre les éléments d'interface 5 à 8 de la pièce d'interface 4 sont définis à partir de scans de patients. Chaque patient présentant des données physiologiques et anatomiques spécifiques, les éventuels écarts sont rattrapés par des pièces souples (collerettes de suture et conduits vasculaires) décrites ci-dessous, qui réalisent la liaison entre le réseau vasculaire du patient et le dispositif de raccordement 1.

Dans un mode de réalisation particulier, la pièce d'interface 4 présente une taille telle qu'elle puisse être inscrite, dans une vue en plan, dans un rectangle de 12 centimètres de longueur et 9 centimètres de largeur.

Le matériau à utiliser pour fabriquer la pièce d'interface 4 doit être suffisamment rigide pour résister aux efforts de fixation. Un matériau métallique tel que l'inox 316L peut notamment être utilisé.

Dans un mode de réalisation préféré, la pièce d'interface 4 est réalisée en titane. Le titane est un matériau biocompatible qui présente des propriétés avantageuses en termes de masse et de tenue mécanique. Ainsi, il est possible de réaliser une pièce d'interface 4 légère, mais suffisamment résistante pour résister aux efforts de fixation des conduits vasculaires (précisés ci-dessous) et de la prothèse cardiaque 2. Une fois la prothèse cardiaque 2 en place, les efforts résultants sont limités.

Une préférence est donnée au titane pur T40 qui présente de bonnes propriétés mécaniques et une excellente biocompatibilité, avec une densité plus faible que l'inox (la masse du dispositif de raccordement 1 devant être limitée pour le patient).

Dans un mode de réalisation préféré, le dispositif de raccordement 1 comporte un conduit vasculaire 14A qui coopère avec l'élément d'interface aortique 7 de la pièce d'interface 4 et un conduit vasculaire 14B qui coopère avec l'élément d'interface pulmonaire 8 de la pièce d'interface 4, comme représenté sur la figure 4.

De préférence, les conduits vasculaires 14A et 14B sont réalisés de façon similaire.

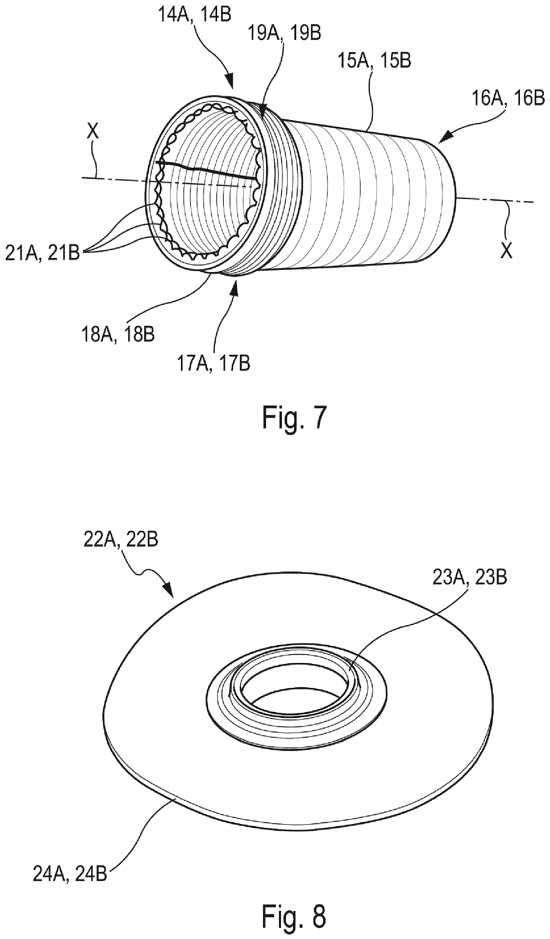

Dans un mode de réalisation préféré représenté sur la figure 7, le conduit vasculaire 14A comporte un élément tubulaire 15A en tissu, d'axe longitudinal X-X, et présente une première extrémité 16A qui est destinée à être suturée sur l'aorte (non représentée) et une seconde extrémité 17A qui est munie d'une bague 18A dite folle, c'est-à-dire qui est montée de façon libre en rotation. Le conduit vasculaire 14A est destiné à être vissé, par l'intermédiaire de cette bague 18A folle, sur l'élément d'interface aortique 7. Grâce à cette bague 18A folle, le conduit vasculaire 14A peut être vissé sur l'élément d'interface aortique 7 en conservant son positionnement (approprié) au cours du vissage. L'élément d'interface aortique 7 comporte un filetage 20A interne comme représenté sur la figure 1, pour permettre le vissage de la bague 18A (pourvue d'un filetage 19A externe) et réaliser ainsi la liaison de l'extrémité 17A du conduit vasculaire 14A à la pièce d'interface 4.

De même, le conduit vasculaire 14B comporte un élément tubulaire 15B en tissu, d'axe longitudinal X-X, et présente, comme représenté sur la figure 7, une première extrémité 16B qui est destinée à être suturée sur l'artère pulmonaire (non représentée) et une seconde extrémité 17B qui est munie d'une bague 18B dite folle, c'est-à-dire qui est montée de façon libre en rotation. Le conduit vasculaire 14B est destiné à être vissé, par l'intermédiaire de cette bague 18B folle, sur l'élément d'interface pulmonaire 8. Grâce à cette bague 18B folle, le conduit vasculaire 14B peut être vissé sur l'élément d'interface aortique 8 en conservant son positionnement (approprié) au cours du vissage. L'élément d'interface pulmonaire 8 comporte un filetage 20B interne comme représenté sur la figure 1, pour permettre le vissage de la bague 18B (pourvue d'un filetage 19B externe) et réaliser ainsi la liaison de l'extrémité 17B du conduit vasculaire 14B à la pièce d'interface 4.

Dans un mode de réalisation préféré, la bague 18A, 18B est réalisée en titane. Elle est suturée sur l'élément tubulaire 15A, 15B du conduit vasculaire 14A, 14B, via des sutures 21A, 21B représentées schématiquement sur la figure 7. On prévoit donc, de préférence, un système de fixation par vissage pour les conduits vasculaires 14A et 14B, qui est adapté au dispositif de raccordement 1.Dans le cadre de la présente invention, un système de fixation autre qu'un vissage peut toutefois également être envisagé.

Lors de l'implantation, l'élément tubulaire 15A, 15B en tissu du conduit vasculaire 14A, 14B est découpé à la longueur appropriée (selon l'axe longitudinal X-X) afin de pouvoir lier le dispositif de raccordement 1 à l'aorte ou à l'artère pulmonaire du patient. Le tissu étant souple, le chirurgien peut de plus adapter son orientation et éviter un phénomène de « kink » via des techniques de suture.

Par ailleurs, le dispositif de raccordement 1 comporte une collerette de suture 22A destinée à être suturée sur l'oreillette gauche du patient et une collerette de suture 22B destinée à être suturée sur l'oreillette droite du patient.

De préférence, les collerettes de suture 22A, 22B sont réalisées de façon similaire.

Chacune desdites collerettes de suture 22A, 22B comporte, comme représenté sur la figure 8, une âme 23A, 23B, de préférence en silicone, qui est configurée pour pouvoir être emboîtée sur l'élément d'interface mitral ou l'élément d'interface tricuspide. Plus précisément, lors de la mise en place de la collerette de suture 22A, 22B, l'âme 23A, 23B est serrée entre la prothèse cardiaque 2 et la pièce d'interface 4. A cet effet, l'âme 23A prend appui sur le plan d'appui P1 montré sur la figure 5, qui est prévu sur la face interne 28A de la pièce d'interface 4, autour de l'orifice 9. De même, l'âme 23B prend appui sur le plan d'appui P2 qui est également prévu sur la face interne 28A de la pièce d'interface 4, autour de l'orifice 10.

Chacune desdites collerettes de suture 22A, 22B, comporte, en plus de l'âme 23A, 23B en silicone, une surface d'interface 24A, 24B destinée à être suturée sur les tissus du patient (oreillettes).

Les collerettes de suture 22A, 22B sont donc utilisées pour relier les oreillettes (gauche et droite) du patient aux éléments d'entrées de la prothèse cardiaque 2, via les éléments d'interface mitral et tricuspide.

Le dispositif de raccordement 1, tel que décrit ci-dessus, est configuré pour pouvoir être installé dans un logement 26 prévu dans le corps de prothèse 3 de la prothèse cardiaque 2, comme représenté sur la figure 6. Le logement 26 présente une forme adaptée et complémentaire de la forme de la face interne 28A (figure 5) de la pièce d'interface 4. En particulier, les plans d'appui P3 et P4 respectivement de l'élément d'interface mitral 5 et de l'élément d'interface tricuspide 6 arrivent au contact de zones correspondent du logement 26. Comme représenté sur la figure 6, le corps de prothèse 3 comprend les ouvertures 9A, 10A, 11A et 12A qui sont destinées à être positionnées en regard respectivement des orifices 9, 10, 11 et 12 de la pièce d'interface 4 lorsque cette dernière est en place sur le corps de prothèse 3.

Pour faciliter la mise en place et le maintien, la pièce d'interface 4 du dispositif de raccordement 1 comporte une languette de centrage 27 longitudinale. Comme représenté sur les figures 4 et 5, cette languette de centrage 27 est saillante par rapport à la surface interne 28A de la plaque plane 13 de la pièce d'interface 4, à une extrémité de la plaque plane 13. Cette languette de centrage 27 est destinée à être insérée dans une gorge 29 allongée (figure 6) pratiquée dans la paroi externe 30 du corps de prothèse 3 de la prothèse cardiaque 2, au niveau du logement 26.

Dans le cadre de la présente invention, le dispositif de raccordement 1 peut être fixé de différentes manières sur la prothèse cardiaque 2, après sa mise en place dans le logement 26, comme illustré sur la figure 2.

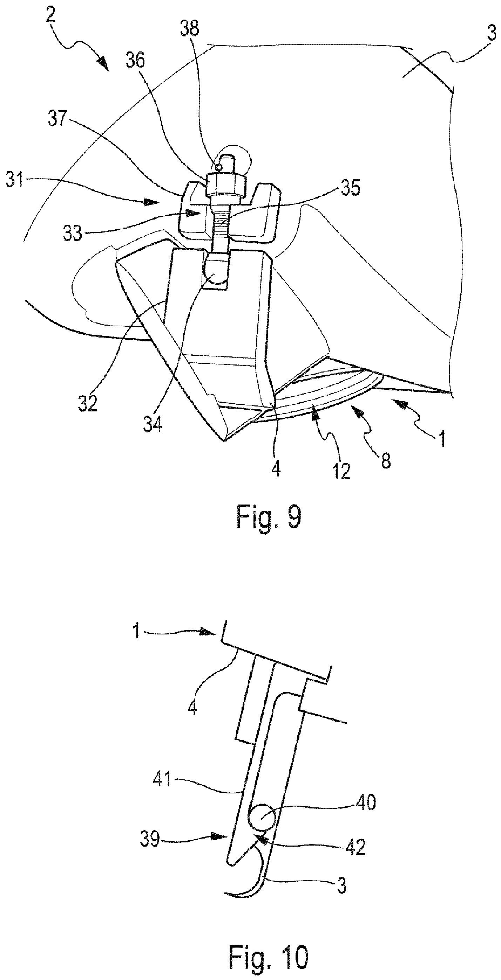

Dans un premier mode de réalisation, la prothèse cardiaque 2 comporte des moyens de fixation 31 par vissage, pour une fixation amovible (par vissage) de la pièce d'interface 4 sur le corps de prothèse 3. Ces moyens de fixation 31 comportent, comme représenté sur la figure 9 :
- une patte 32 solidaire de la pièce d'interface 4, qui est pourvue d'une goupille 33. Cette goupille 33 est munie à une première de ses extrémités d'une tête 34 montée mobile dans un siège prévu dans la patte 32, et elle est pourvue d'un filetage 35 à l'autre extrémité (libre), destiné à recevoir un écrou 36 ; et
- une plaquette d'appui 37 solidaire du corps de prothèse 3, qui est destinée à coopérer avec l'extrémité libre de la goupille 33.

Le mode de vissage consiste, après la mise en place du dispositif de raccordement 1 sur le corps de prothèse 3, à rabattre la goupille 33 sur le corps de prothèse 3 et à visser l'écrou 36 intégré pour le maintenir contre la plaquette d'appui 37, afin de verrouiller le dispositif de raccordement 1. Une cheville de sécurité 38 peut être mise en place pour empêcher un dévissage non souhaité. Une fois la prothèse cardiaque 2 implantée, la zone destinée au vissage est visible et facilement accessible pour le chirurgien.

En outre, dans un second mode de réalisation, la prothèse cardiaque 2 comporte des moyens de fixation 39 par clipsage pour la fixation amovible (par clipsage) de la pièce d'interface 4 sur le corps de prothèse 3. Ces moyens de fixation 39 comportent, comme représenté sur la figure 10, une goupille 40 (ou surépaisseur sur le corps de prothèse 3) intégrée à la prothèse cardiaque 2 et une patte de fixation 41 solidaire de la pièce d'interface 4. Cette patte de fixation 41 est pourvue d'un crochet 42 à son extrémité libre et elle est légèrement déformable pour pouvoir être accrochée via le crochet 42 par un léger effort à la goupille 40 et se bloquer une fois l'effort relâché.

La pièce d'interface 4, telle que décrite ci-dessus, présente les caractéristiques avantageuses suivantes :
- elle est biocompatible ;
- elle est implantable pour une longue durée ;
- elle est nettoyable et apte à être stérilisée ;
- elle garantit l'étanchéité avec la prothèse cardiaque 2 ; et
- elle est pourvue d'un mode de fixation ergonomique et rapide (qui permet de limiter la durée de la circulation extracorporelle).

La pièce d'interface 4 est donc destinée à être liée :
- d'un côté, aux oreillettes (droite et gauche) qui sont préparées au préalable (avec la suture des collerettes de suture 22A et 22B), ainsi qu'aux conduits vasculaires 14A et 14B qui sont suturés sur les artères (aorte et artère pulmonaire) ; et
- de l'autre côté, directement à la prothèse cardiaque 2 pourvue du logement 26 dans lequel vient s'adapter le dispositif de raccordement 1, pour compléter la surface au contact des tissus.

On décrit ci-après les étapes principales de la mise en place du dispositif de raccordement 1 et de la prothèse cardiaque 2.

Après ablation des ventricules gauche et droit du patient, les collerettes de suture 22A et 22B sont suturées sur les oreillettes gauche et droite du patient. Puis, la pièce d'interface 4 est amenée sur les oreillettes. Le plan auriculaire forme un plan de référence relativement rigide qui va permettre à la pièce d'interface 4 (dont les éléments d'interface présentent les distances et les orientations appropriées précisées ci-dessus) de se positionner correctement vis-à-vis des artères.

Lorsque la pièce d'interface 4 est placée sur les oreillettes, l'orientation des conduits vasculaires 14A et 14B et la distance pour couper ces conduits vasculaires 14A et 14B à la longueur appropriée sont naturellement définies. La pièce d'interface 4 étant adaptée à la prothèse cardiaque 2 pour une fixation rapide, les orientations de la prothèse cardiaque 2 sont déjà respectées et les sutures ne seront pas sollicitées lors de sa mise en place.

Chacun des conduits vasculaires 14A et 14B est alors coupé à la longueur appropriée.

Chacun des conduits vasculaires 14A et 14B est vissé via son extrémité 17A, 17B sur l'élément d'interface 7, 8 correspondant de la pièce d'interface 4, à l'aide de la bague 18A, 18B intégrée au conduit vasculaire 14A, 14B et est suturé via son autre extrémité 16A, 16B à l'artère correspondante (aorte, artère pulmonaire).

Les conduits vasculaires 14A et 14B sont donc mis en place en l'absence du corps de prothèse 3, ce qui facilite le travail du chirurgien. De plus, un seul chirurgien peut réaliser ces opérations.

Une fois les sutures effectuées et le dispositif de raccordement 1 mis en place, la prothèse cardiaque 2 est montée sur le dispositif de raccordement 1 et est fixée à ce dernier. Pour le montage, la pièce d'interface 4 est placée dans le logement 26 prévu à cet effet dans la paroi externe 30 du corps de prothèse. Pour la fixation, on réalise un vissage ou un clipsage, en fonction du mode de réalisation retenu pour les moyens de fixation, comme indiqué ci-dessus.

On est ainsi en mesure de fixer la prothèse cardiaque 2 sur une pièce d'interface 4 (rigide) qui respecte l'anatomie du patient, via :
- le respect de l'écartement moyen des oreillettes ;
- la découpe des conduits vasculaires à la bonne longueur ;
- le respect de l'orientation moyenne des conduits vasculaires ; et
- la réalisation de la suture en fonction de zones de renfort à privilégier (permettant de limiter le phénomène de « kink »).

La pièce d'interface 4 permet donc de préparer le patient avant la mise en place de la prothèse cardiaque 2 de manière ergonomique. Outre le gain de temps généré par cette solution, l'utilisation d'ancillaires d'implantation n'est plus nécessaire pour la mise en place des conduits vasculaires 14A et 14B, le dispositif de raccordement 1 répondant déjà au besoin. Par conséquent, moins de pièces sont à fournir, et donc moins de pièces sont utilisées au bloc opératoire, ce qui génère également un gain de coût. La pièce d'interface 4 sert ainsi à la fois d'ancillaire d'implantation et d'élément de la prothèse cardiaque 2 implantée.

Le dispositif de raccordement 1 et la prothèse cardiaque 2, tels que décrits ci-dessus, présentent donc de nombreux avantages. En particulier, ils permettent d'obtenir :
- une amélioration de l'ergonomie du chirurgien ;
- une réduction du nombre de pièces implantées (pas d'agrafes notamment) ;
- une réduction du nombre de pièces (ou ancillaires) nécessaires à l'implantation ; et
- une réduction de la durée d'implantation.

## Revendications

1. Dispositif de raccordement, ledit dispositif de raccordement (1) étant destiné au raccordement d'une prothèse cardiaque (2) implantable dans la cavité péricardique d'un patient au réseau vasculaire du patient, ladite prothèse cardiaque (2) étant apte à remplacer les ventricules gauche et droit naturels dudit patient après ablation de ces derniers,
**caractérisé en ce qu'**il comporte une unique pièce d'interface (4) configurée pour pouvoir être fixée à la prothèse cardiaque (2), la pièce d'interface (4) étant munie d'un élément d'interface dit mitral (5), d'un élément d'interface dit tricuspide (6), d'un élément d'interface dit aortique (7) et d'un élément d'interface dit pulmonaire (8) destinés, respectivement, au raccordement à l'oreillette gauche, à l'oreillette droite, à l'aorte et à l'artère pulmonaire du patient, chacun desdits éléments d'interface (5 à 8) étant pourvu d'un orifice (9 à 12), et lesdits éléments d'interface (5 à 8) présentant des orientations (5A à 8A) prédéterminées.

2. Dispositif selon la revendication 1,
**caractérisé en ce que** l'orientation de l'élément d'interface mitral (5) et l'orientation de l'élément d'interface tricuspide (6) présentent entre elles un angle compris entre 0 °et 32°.

3. Dispositif selon la revendication 1,
**caractérisé en ce que** l'élément d'interface mitral (5) et l'élément d'interface tricuspide (6) sont agencés sur une plaque plane (13) de la pièce d'interface (4).

4. Dispositif selon l'une quelconque des revendications précédentes, **caractérisé en ce que** la pièce d'interface (4) présente au moins certaines des orientations suivantes :
- l'orientation (7A) de l'élément d'interface aortique (7) et l'orientation (5A) de l'élément d'interface mitral (5) présentent, entre elles, un angle compris entre 0° et 90° ;
- l'orientation (7A) de l'élément d'interface aortique (7) et l'orientation (6A) de l'élément d'interface tricuspide (6) présentent, entre elles, un angle compris entre 0° et 92° ;
- l'orientation (8A) de l'élément d'interface pulmonaire (8) et l'orientation (5A) de l'élément d'interface mitral (5) présentent, entre elles, un angle compris entre 26° et 64° ;
- l'orientation (8A) de l'élément d'interface pulmonaire (8) et l'orientation (6A) de l'élément d'interface tricuspide (6) présentent, entre elles, un angle compris entre 31° et 73° ;
- l'orientation (7A) de l'élément d'interface aortique (7) et l'orientation (8A) de l'élément d'interface pulmonaire (8) présentent, entre elles, un angle compris entre 39° et 79°.

5. Dispositif selon l'une quelconque des revendications précédentes, **caractérisé en ce que** la pièce d'interface (4) présente au moins certaines des distances suivantes :
- la distance entre le centre (5B) de l'orifice (9) de l'élément d'interface mitral (5) et le centre (6B) de l'orifice (10) de l'élément d'interface tricuspide (6) est comprise entre 42 mm et 76 mm ;
- la distance entre le centre (5B) de l'orifice (9) de l'élément d'interface mitral (5) et le centre (7B) de l'orifice (11) de l'élément d'interface aortique (7) est comprise entre 37 mm et 65 mm ;
- la distance entre le centre (5B) de l'orifice (9) de l'élément d'interface mitral (5) et le centre (8B) de l'orifice (12) de l'élément d'interface pulmonaire (8) est comprise entre 50 mm et 80 mm ;
- la distance entre le centre (6B) de l'orifice (10) de l'élément d'interface tricuspide (6) et le centre (7B) de l'orifice (11) de l'élément d'interface aortique (7) est comprise entre 34 mm et 64 mm ;
- la distance entre le centre (6B) de l'orifice (10) de l'élément d'interface tricuspide (6) et le centre (8B) de l'orifice (12) de l'élément d'interface pulmonaire (8) est comprise entre 59 mm et 91 mm ;
- la distance entre le centre (7B) de l'orifice (11) de l'élément d'interface aortique (7) et le centre (8B) de l'orifice (12) de l'élément d'interface pulmonaire (8) est comprise entre 22 mm et 46 mm.

6. Dispositif selon l'une quelconque des revendications précédentes, **caractérisé en ce que** l'élément d'interface aortique (7) et l'élément d'interface pulmonaire (8) comportent, chacun, un filetage (20A, 20B).

7. Dispositif selon l'une quelconque des revendications précédentes, **caractérisé en ce que** la pièce d'interface (4) est réalisée en l'un des matériaux suivants : du titane, de l'inox.

8. Dispositif selon l'une quelconque des revendications précédentes, **caractérisé en ce qu'**il comporte de plus :
- un premier conduit vasculaire (14A) dont une première extrémité (16A) est destinée à être suturée sur l'aorte du patient et dont la seconde extrémité (17A) est munie d'une bague (18A) montée de façon libre en rotation et apte à être vissée sur l'élément d'interface aortique (7) ; et
- un second conduit vasculaire (14B) dont une première extrémité (16B) est destinée à être suturée sur l'artère pulmonaire du patient et dont la seconde extrémité (17B) est munie d'une bague (18B) montée de façon libre en rotation et apte à être vissée sur l'élément d'interface pulmonaire (8).

9. Dispositif selon l'une quelconque des revendications précédentes, **caractérisé en ce qu'**il comporte de plus :
- une première collerette de suture (22A) destinée à être suturée sur l'oreillette gauche du patient et comportant une âme (23A) configurée pour pouvoir être montée sur l'élément d'interface mitral (5) ; et
- une seconde collerette de suture (22B) destinée à être suturée sur l'oreillette droite du patient et comportant une âme (23B) configurée pour pouvoir être montée sur l'élément d'interface tricuspide (6).

10. Prothèse cardiaque implantable dans la cavité péricardique d'un patient, ladite prothèse cardiaque (2) étant apte à remplacer les ventricules gauche et droit naturels dudit patient après ablation de ces derniers, **caractérisée en ce qu'**elle comporte au moins un corps de prothèse (3) rigide dans lequel sont agencés au moins des ventricules artificiels gauche et droit, et un dispositif de raccordement (1) selon l'une quelconque des revendications 1 à 9, configuré pour pouvoir être fixé dans un logement (26) prévu dans le corps de prothèse (3).

11. Prothèse cardiaque selon la revendication 10,
**caractérisée en ce que** la pièce d'interface (4) du dispositif de raccordement (1) comporte une languette de centrage (27) qui coopère avec une gorge (29) pratiquée dans la paroi externe (30) du corps de prothèse (3).

12. Prothèse cardiaque l'une des revendications 10 et 11, **caractérisée en ce qu'**elle comporte des moyens de fixation par vissage (31) pour une fixation amovible de la pièce d'interface (4) sur le corps de prothèse (3).

13. Prothèse cardiaque selon l'une des revendications 10 et 11, **caractérisée en ce qu'**elle comporte des moyens de fixation par clipsage (39) pour une fixation amovible de la pièce d'interface (4) sur le corps de prothèse (3).

## Patentansprüche

1. Vorrichtung zur Verbindung, wobei die Vorrichtung (1) zur Verbindung einer in die Perikardhöhle eines Patienten implantierbaren Herzprothese (2) mit dem Gefäßsystem des Patienten bestimmt ist, wobei die Herzprothese (2) in der Lage ist, das linke und rechte natürliche Ventrikel des Patienten nach Ablation von diesen zu ersetzen,
**dadurch gekennzeichnet, dass** sie ein einzigartiges Schnittstellenstück (4) umfasst, das konfiguriert ist, um an der Herzprothese (2) befestigt werden zu können, wobei das Schnittstellenstück (4) mit einem Mitralschnittstellenelement (5), einem Trikuspidschnittstellenelement (6), einem Aortaschnittstellenelement und einem Lungenschnittstellenelement (8) ausgestattet ist, die jeweils zur Verbindung mit dem linken Vorhof, mit dem rechten Vorhof, mit der Aorta und mit der Lungenarterie des Patienten bestimmt sind, wobei jedes der Schnittstellenelemente (5 bis 8) mit einer Öffnung (9 bis 12) versehen ist und die Schnittstellenelemente (5 bis 8) vorgegebene Orientierungen (5A bis 8A) aufweisen.

2. Vorrichtung nach Anspruch 1,
**dadurch gekennzeichnet, dass** die Orientierung des Mitralschnittstellenelements (5) und die Orientierung des Trikuspidschnittstellenelements (6) untereinander einen Winkel zwischen 0° und 32° aufweisen.

3. Vorrichtung nach Anspruch 1,
**dadurch gekennzeichnet, dass** das Mitralschnittstellenelement (5) und das Trikuspidschnittstellenelement (6) auf einer ebenen Platte (13) des Schnittstellenstücks (4) angeordnet sind.

4. Vorrichtung nach einem der vorstehenden Ansprüche,
**dadurch gekennzeichnet, dass** das Schnittstellenstück (4) mindestens gewisse der folgenden Orientierungen aufweist:
- die Orientierung (7A) des Aortaschnittstellenelements (7) und die Orientierung (5A) des Mitralschnittstellenelements (5) weisen untereinander einen Winkel zwischen 0° und 90° auf;
- die Orientierung (7A) des Aorataschnittstellenelements (7) und die Orientierung (6A) des Trikuspidschnittstellenelements (6) weisen untereinander einen Winkel zwischen 0°und 92° auf;
- die Orientierung (8A) des Lungenschnittstellenelements (8) und die Orientierung (5A) des Mitralschnittstellenelements (5) weisen untereinander einen Winkel zwischen 26° und 64° auf;
- die Orientierung (8A) des Lungenschnittstellenelements (8) und die Orientierung (6A) des Trikuspidschnittstellenelements (6) weisen untereinander einen Winkel zwischen 31° und 73° auf;
- die Orientierung (7A) des Aortaschnittstellenelements (7) und die Orientierung (8A) des Lungenschnittstellenelements (8) weisen untereinander einen Winkel zwischen 39° und 79° auf.

5. Vorrichtung nach einem der vorstehenden Ansprüche,
**dadurch gekennzeichnet, dass** das Schnittstellenstück (4) mindestens gewisse der folgenden Abstände aufweist:
- der Abstand zwischen dem Mittelpunkt (5B) der Öffnung (9) des Mitralschnittstellenelements (5) und dem Mittelpunkt (6B) der Öffnung (10) des Trikuspidschnittstellenelements (6) beträgt zwischen 42 mm und 76 mm;
- der Abstand zwischen dem Mittelpunkt (5B) der Öffnung (9) des Mitralschnittstellenelements (5) und dem Mittelpunkt (7B) der Öffnung (11) des Aortaschnittstellenelements (7) beträgt zwischen 37 mm und 65 mm;
- der Abstand zwischen dem Mittelpunkt (5B) der Öffnung (9) des Mitralschnittstellenelements (5) und dem Mittelpunkt (8B) der Öffnung (12) des Lungenschnittstellenelements (8) beträgt zwischen 50 mm und 80 mm;
- der Abstand zwischen dem Mittelpunkt (6B) der Öffnung (10) des Trikuspidschnittstellenelements (6) und dem Mittelpunkt (7B) der Öffnung (11) des Aortaschnittstellenelements (7) beträgt zwischen 34 mm und 64 mm;
- der Abstand zwischen dem Mittelpunkt (6B) der Öffnung (10) des Trikuspidschnittstellenelements (6) und dem Mittelpunkt (8B) der Öffnung (12) des Lungenschnittstellenelements (8) beträgt zwischen 59 mm und 91 mm;
- der Abstand zwischen dem Mittelpunkt (7B) der Öffnung (11) des Aortaschnittstellenelements (7) und dem Mittelpunkt (8B) der Öffnung (12) des Lungenschnittstellenelements (8) beträgt zwischen 22 mm und 46 mm.

6. Vorrichtung nach einem der vorstehenden Ansprüche,
**dadurch gekennzeichnet, dass** das Aortaschnittstellenelement (7) und das Lungenschnittstellenelement (8) jeweils ein Gewinde (20A, 20B) umfassen.

7. Vorrichtung nach einem der vorstehenden Ansprüche,
**dadurch gekennzeichnet, dass** das Schnittstellenstück (4) aus einem der folgenden Materialien hergestellt ist: Titan, Edelstahl.

8. Vorrichtung nach einem der vorstehenden Ansprüche,
**dadurch gekennzeichnet, dass** sie außerdem Folgendes umfasst:
- ein erstes Gefäßrohr (14A), von dem ein erstes Ende (16A) dazu bestimmt ist, an der Aorta des Patienten vernäht zu werden, und von dem das zweite Ende (17A) mit einem Ring (18A) ausgestattet ist, der auf frei rotierende Art montiert und in der Lage ist, auf das Aortaschnittstellenelement (7) geschraubt zu werden; und
- ein zweites Gefäßrohr (14B), von dem ein erstes Ende (16B) dazu bestimmt ist, an der Lungenarterie des Patienten vernäht zu werden, und von dem das zweite Ende (17B) mit einem Ring (18B) ausgestattet ist, der auf frei rotierende Art montiert und in der Lage ist, auf das Lungenschnittstellenelement (8) geschraubt zu werden.

9. Vorrichtung nach einem der vorstehenden Ansprüche,
**dadurch gekennzeichnet, dass** sie außerdem Folgendes umfasst:
- einen ersten Nahtkragen (22A), der dazu bestimmt ist, an dem linken Vorhof des Patienten vernäht zu werden und einen Steg (23A) umfasst, der konfiguriert ist, um an dem Mitralschnittstellenelement (5) montiert werden zu können; und
- einen zweiten Nahtkragen (22B), der dazu bestimmt ist, an dem rechten Vorhof des Patienten vernäht zu werden und einen Steg (23B) umfasst, der konfiguriert ist, um an dem Trikuspidschnittstellenelement (6) montiert werden zu können.

10. In die Perikardhöhle eines Patienten implantierbare Herzprothese, wobei die Herzprothese (2) in der Lage ist, das linke und rechte natürliche Ventrikel des Patienten nach der Ablation von diesen zu ersetzen,
**dadurch gekennzeichnet, dass** sie mindestens einen starren Prothesenkörper (3) umfasst, in dem mindestens das künstliche linke und rechte Ventrikel angeordnet sind, und eine Vorrichtung zur Verbindung (1) nach einem der Ansprüche 1 bis 9, die konfiguriert ist, um an einer in dem Prothesenkörper (3) vorgesehenen Aufnahme (26) befestigt zu werden.

11. Herzprothese nach Anspruch 10,
**dadurch gekennzeichnet, dass** das Schnittstellenstück (4) der Vorrichtung zur Verbindung (1) eine Zentrierlasche (27) umfasst, die mit einer in der externen Wand (30) des Prothesenkörpers (3) praktizierten Vertiefung (29) zusammenwirkt.

12. Herzprothese nach einem der Ansprüche 10 und 11,
**dadurch gekennzeichnet, dass** sie Befestigungsmittel durch Verschraubung (31) für eine austauschbare Befestigung des Schnittstellenstücks (4) an dem Prothesenkörper (3) umfasst.

13. Herzprothese nach einem der Ansprüche 10 und 11,
**dadurch gekennzeichnet, dass** sie Befestigungsmittel durch Schnappverbindung (39) für eine austauschbare Befestigung des Schnittstellenstücks (4) an dem Prothesenkörper (3) umfasst.

## Claims

1. A connecting device, said connecting device (1) being intended for connecting a heart prosthesis (2) implantable in the pericardial cavity of a patient to the vascular system of the patient, said heart prosthesis (2) being capable of replacing the natural left and right ventricles of said patient after ablation of the latter,
**characterised in that** it comprises a single interface part (4) configured to be able to be attached to the heart prosthesis (2), the interface part (4) being equipped with an interface element referred to as mitral interface element (5), an interface element referred to as tricuspid interface element (6), an interface element referred to as aortic interface element (7) and an interface element referred to as pulmonary interface element (8) intended, respectively, for the connection to the left atrium, to the right atrium, to the aorta and to the pulmonary artery of the patient, each of said interface elements (5 to 8) being provided with an orifice (9 to 12), and said interface elements (5 to 8) having predetermined orientations (5A to 8A).

2. The device according to claim 1,
**characterised in that** the orientation of the mitral interface element (5) and the orientation of the tricuspid interface element (6) have an angle between them of between 0° and 32°.

3. The device according to claim 1,
**characterised in that** the mitral interface element (5) and the tricuspid interface element (6) are arranged on a flat plate (13) of the interface part (4).

4. The device according to any one of the preceding claims,
**characterised in that** the interface part (4) has at least some of the following orientations:
- the orientation (7A) of the aortic interface element (7) and the orientation (5A) of the mitral interface element (5) have, between them, an angle of between 0° and 90°;
- the orientation (7A) of the aortic interface element (7) and the orientation (6A) of the tricuspid interface element (6) have, between them, an angle of between 0° and 92°;
- the orientation (8A) of the pulmonary interface element (8) and the orientation (5A) of the mitral interface element (5) have, between them, an angle of between 26° and 64°;
- the orientation (8A) of the pulmonary interface element (8) and the orientation (6A) of the tricuspid interface element (6) have, between them, an angle of between 31° and 73°;
- the orientation (7A) of the aortic interface element (7) and the orientation (8A) of the pulmonary interface element (8) have, between them, an angle of between 39° and 79°.

5. The device according to any one of the preceding claims,
**characterised in that** the interface part (4) has at least some of the following distances:
- the distance between the centre (5B) of the orifice (9) of the mitral interface element (5) and the centre (6B) of the orifice (10) of the tricuspid interface element (6) is between 42 mm and 76 mm;
- the distance between the centre (5B) of the orifice (9) of the mitral interface element (5) and the centre (7B) of the orifice (11) of the aortic interface element (7) is between 37 mm and 65 mm;
- the distance between the centre (5B) of the orifice (9) of the mitral interface element (5) and the centre (8B) of the orifice (12) of the pulmonary interface element (8) is between 50 mm and 80 mm;
- the distance between the centre (6B) of the orifice (10) of the tricuspid interface element (6) and the centre (7B) of the orifice (11) of the aortic interface element (7) is between 34 mm and 64 mm;
- the distance between the centre (6B) of the orifice (10) of the tricuspid interface element (6) and the centre (8B) of the orifice (12) of the pulmonary interface element (8) is between 59 mm and 91 mm;
- the distance between the centre (7B) of the orifice (11) of the aortic interface element (7) and the centre (8B) of the orifice (12) of the pulmonary interface element (8) is between 22 mm and 46 mm.

6. The device according to any one of the preceding claims,
**characterised in that** the aortic interface element (7) and the pulmonary interface element (8) each comprise a thread (20A, 20B).

7. The device according to any one of the preceding claims,
**characterised in that** the interface part (4) is made of one of the following materials: titanium, stainless steel.

8. The device according to any one of the preceding claims,
**characterised in that** it further comprises:
- a first vascular conduit (14A), a first end (16A) of which is intended to be sutured to the aorta of the patient and the second end (17A) of which is equipped with a ring (18A) mounted so as to rotate freely and able to be screwed onto the aortic interface element (7); and
- a second vascular conduit (14B), a first end (16B) of which is intended to be sutured to the pulmonary artery of the patient and the second end (17B) of which is equipped with a ring (18B) mounted so as to rotate freely and capable of being screwed onto the pulmonary interface element (8).

9. The device according to any one of the preceding claims,
**characterised in that** it further comprises:
- a first suture collar (22A) intended to be sutured to the left atrium of the patient and comprising a core (23A) configured so that it can be mounted on the mitral interface element (5); and
- a second suture collar (22B) intended to be sutured to the right atrium of the patient and comprising a core (23B) configured so that it can be mounted on the tricuspid interface element (6).

10. A heart prosthesis implantable in the pericardial cavity of a patient, said heart prosthesis (2) being capable of replacing the natural left and right ventricles of said patient after ablation of the latter,
**characterised in that** it comprises at least one rigid prosthesis body (3) in which at least left and right artificial ventricles are arranged, and a connecting device (1) according to any one of claims 1 to 9, configured so that it can be attached in a housing (26) provided in the prosthesis body (3).

11. The heart prosthesis as claimed in claim 10,
**characterised in that** the interface part (4) of the connecting device (1) comprises a centring tongue (27) which cooperates with a gorge (29) fitted in the external wall (30) of the prosthesis body (3).

12. The heart prosthesis according to one of claims 10 and 11,
**characterised in that** it comprises screw attachment means (31) for removably attaching the interface part (4) to the prosthesis body (3).

13. The heart prosthesis according to one of claims 10 and 11,
**characterised in that** it comprises clip-attachment means (39) for removably attaching the interface part (4) to the prosthesis body (3).
